# EUROPEAN PATENT SPECIFICATION

(11) **EP 2 588 488 B1**
(45) Date of publication and mention of the grant of the patent: **15.03.2017**
(21) Application number: 10747311.8
(22) Date of filing: 30.06.2010
(51) Int. Cl.: C07K 1/107, C07K 14/47

(54) **IRON (III) CASEINSUCCINYLATE AND METHOD FOR THE PREPARATION THEREOF**
EISEN (III)-CASEINSUCCINYLAT UND VERFAHREN ZU SEINER HERSTELLUNG
CASÉINESUCCINYLATE DE FER (III) ET SON PROCÉDÉ DE PRÉPARATION

(43) Date of publication of application: 08.05.2013
(73) Proprietor: Italfarmaco SpA, 20126 Milano (IT)
(72) Inventor: STEVENAZZI, Andrea, I-20030 Bovisio Masciago (MB) (IT); DE FERRA, Lorenzo, I-00162 Roma (IT); PINTO, Barbara, I-00125 Roma (IT)
(74) Representative: Pistolesi, Roberto
(86) International application number: PCT/IT2010/000287
(87) International publication number: WO 2012/001712

(56) References cited:
- EP-A1- 0 319 664
- EP-A2- 0 939 083
- WO-A1-2006/021843
- WO-A1-2007/065812
- GB-A- 2 115 821
- CREMONESI P ET AL: "IRON DERIVATIVES OF MODIFIED MILK PROTEIN", ARZNEIMITTEL FORSCHUNG. DRUG RESEARCH, ECV EDITIO CANTOR VERLAG, AULENDORF, DE, vol. 34, no. 9, 1 January 1984 (1984-01-01), pages 948-952, XP001207730, ISSN: 0004-4172 cited in the application

## Description

The present invention refers to an iron (III) caseinsuccinylate characterised by a content of iron comprised between 4.5% and 7% by weight and by a solubility in water greater than about 92% and by having a phosphorous/nitrogen ratio greater than 5% by weight.

The present invention further refers to a method for preparing iron (III) caseinsuccinylate.

### PRIOR ART

Derivatives of iron are widely used in medicine for prevention and cure of anaemia and pregnancy iron deficiencies, in cases of malabsorption syndrome, during breastfeeding and growth. Among these, Iron Protein Succinilate commonly referred to by the acronym IPS is positively distinguished for the bioavailability and tolerability characteristics thereof.

Chemical, physical and biological properties as well as the preparation of IPS, called Ironlat, obtained starting from milk proteins are described in the Italian patent IT1150213. Furthermore, the solubility of Ironlat is indicated at pH>5 which is partial for the complex containing 6.7% of iron and complete for the one containing 4.6%.

Cremonesi et al. in International Journal of Clinical Pharmacology Therapy and Toxicology vol.31 (1993) pages 40-51 indicates the chemical and pharmacological properties of iron (III) caseinsuccinylate, complex of iron (III) obtained starting from succinylated casein. In the in vivo experimentation, this product, having an iron content equivalent to 5%, reveals a better gastrointestinal tolerability with respect to other derivatives of iron.

European patent EP939083B1 describes a method for producing iron (III) caseinsuccinylate which provides for the use of specific dilaceration and drying procedures to overcome the problem of the presence of insoluble components in the product. Furthermore, described is the use of food grade casein as raw material to be used in the process of producing iron (III) caseinsuccinylate.

In order to contribute to keeping the product within specified limits regarding microbiological contamination, paraben methyl and paraben propyl preservatives are added during the process; presence at levels of 3% and 1.05% are respectively determined when analyzing the finished product. Such use of preservatives is also described in patent EP1776382B1 which introduces the use of moist granulation in combination with spray drying or lyophilisation of the product to facilitate, during the process, the solubilisation processes.

Patent application EP319664 proposes a method based on enzymatic degradation of iron (III) caseinsuccinylate to overcome the problems of solubility and viscosity observed in this complex, in particular when the specific value of iron exceeds 10%.

International patent application WO2007/065812 describes the preparation of iron (III) caseinsuccinylate performing both the succinylation reaction and the subsequent complexation using the iron (III) ion operating in suspension, while the aspects regarding the difficulties of solubilising iron (III) caseinsuccinylate are not tackled. Actually, this patent application does not provide information regarding the solubilisation of the obtained complex, and the viscosity of the possibly obtainable solution is not measured.

Such solubilisation problems are particularly apparent when the specific iron content exceeds the limit of 5%.

The viscosity increase phenomenon, which is observed as the concentration of the product in the aqueous solution increases, makes it difficult, or even hinders the use of iron (III) caseinsuccinylate beyond determined concentrations.

The combination of these effects limits the dosage of the product to a value not exceeding 800 mg corresponding to about 40 mg as iron (III) per dose unless using formulations exceeding 15 ml of solution.

Furthermore, the viscosity of an aqueous solution of iron (III) caseinsuccinylate complicates the operations of reducing the microbial content of the product through filtration or microfiltration, and requires the use of high amounts of preservatives.

It is thus apparent how the current art does not offer simple solutions for overcoming the solubilisation problems of iron (III) caseinsuccinylate, a drug highly at treating various forms of iron deficiency.

### DESCRIPTION OF THE INVENTION

It has now been discovered that surprising advantages may be obtained in the production of iron (III) caseinsuccinylate if dietary casein - which has been subjected to a purification process aimed at removing protein impurities, inorganic impurities and/or to increase the phosphorous/nitrogen ratio (P/N ratio) - is used as raw material. As a matter of fact, it is thus possible to obtain iron (III) caseinsuccinylate not only characterised by a protein profile containing lower impurities but unexpectedly also by a an improved solubility in water. Such improved solubility entails the possibility to obtain aqueous solutions and/or suspensions revealing lower viscosity with respect to that obtained starting from commercial food grade casein.

Referring to casein, the expression "protein impurities" according to the invention is used to indicate proteins different from casein in its various variants, measured by means of one dimensional electrophoresis with Coomassie Blue staining and densitometric analysis. Preferably, said protein impurities of the invention are proteins different from alpha-S1 casein, alpha-S2 casein, beta casein, kappa casein and/or pseudo-kappa casein.

Analogously, referring to iron (III) caseinsuccinylate, the expression "protein impurities" according to the invention is used to indicate substances different from succinylated casein in its various variants, measured by means of one dimensional electrophoresis with Coomassie Blue staining and densitometric analysis. Preferably said protein impurities of the invention are succinylated proteins different from the products of succinylation of alpha-S1 casein, alpha-S2 casein, beta casein, kappa casein and/or pseudo-kappa casein.

The expression "inorganic impurities" according to the invention is used to indicate sulphated ashes.

The term "P/N ratio" is used to indicate the ratio between the amount by weight of phosphorous present in the sample, measured by means of the ICP technique, and the amount by weight of nitrogen, measured through elementary analysis.

According to the present invention the values expressed in percentage by weight (% by weight) shall be intended with respect to the total weight of iron (III) caseinsuccinylate or of casein.

Thus, the present invention has the object of iron (III) caseinsuccinylate characterised by a content of iron comprised between 4.5 and 7% by weight and a solubility in water greater than about 92% and by having a phosphorous/nitrogen ratio greater than 5% by weight. Preferably, such solubility in water is to be considered as solubility in about 11.5 parts of water.

Said iron (III) caseinsuccinylate further contains an amount of protein impurities lower than about 15%, preferably lower than about 10% and/or a P/N ratio greater than 5%, preferably greater than about 5.5%.

Further object of the present invention is a method for preparing iron (III) caseinsuccinylate described above, comprising the following steps:
a) reacting casein and succinic anhydride in water to obtain succinylated casein,
b) reaction of succinylated casein with iron (III) chloride to obtain iron (III) caseinsuccinylate,
characterised in that said casein according to step a) is purified casein, having a low protein impurities content and/or inorganic impurities and/or a high P/N ratio.

Preferably, said protein impurities content is lower than 15% by weight, that of said inorganic impurities is lower than 1% by weight and the ratio between content by weight of phosphorous and the content by weight of nitrogen is greater than 5% by weight.

More preferably, the content of said protein impurities is lower than about 10% by weight and/or said inorganic impurities content is lower than about 0.4% by weight and/or the ratio between content by weight of phosphorous and the content by weight of nitrogen is greater than about 5.5%.

Said protein impurities of the invention are proteins different from alpha-S1 casein, alpha-S2 casein, beta casein, kappa casein and/or pseudo-kappa casein.

Dietary caseins are generally identified according to the precipitation technique employed in the preparation thereof: regarding acid caseins precipitation is performed by means of acidification, while those obtained using rennet are presamic, also commonly referred to as rennet caseins.

According to the present invention, the dietary casein used as raw material may be acid casein or presamic casein.

These commercial products, regardless of the type (acid or presamic) are usually accompanied by scarce analytic documentation; the parameters typically indicated are the water content, the protein content (determined by applying a multiplication factor to the total nitrogen value), the level of microbial contamination and the granulometry. This level of characterisation is fully suitable for the use of caseins in the food industry, but we have now discovered that the preparation of iron (III) caseinsuccinylate requires an in-depth analytic evaluation of this raw material.

Commercial dietary caseins and purified caseins used as raw materials according to the present invention for the preparation of iron (III) caseinsuccinylate were analysed by means of Reverse Phase HPLC according to the method indicated in Bonizzi et al. Journal of Chromatography A vol.1261(2009) pages 165-168.

It is thus possible to identify three types of caseins: alpha-S1, alpha-S2, beta, kappa and pseudokappa.

Through electrophoresis analysis, both mono-dimensional and bi-dimensional, using Coomassie Blue as a reagent indicator, alongside the separation of various types of casein and the various varieties thereof deriving from their post-transcriptional modifications (such as glycosylation and phosphorylation), it is also possible to evaluate the presence of the protein impurities.

In particular, SDS-PAGE electrophoresis with 14% polyacrylamide gel, staining with Coomassie Blue and densitometric analysis based on a calibration line constructed with different amounts of BSA, was used for quantitative determination of the protein impurities.

Said caseins and purified caseins were also analysed using the sulphated ash method according to Farmacopea Europea (method Eu.Pharm. 2.4.14). The phosphorous (P) content thereof was then determined through the ICP technique and the Nitrogen (N) content was determined through elementary analysis and thus the P/N % ratio was calculated by dividing the amount by weight observed regarding phosphorous by the amount by weight observed regarding nitrogen and multiplying the result by 100. The table 1 below shows the results obtained for the various types of caseins, i.e. commercial dietary caseins (acid and presamic) and the purified caseins according to the method of the present invention.

**Table 1**

| Casein | Raw material for | Protein impurities | Sulphated ashes | P/N % |
|---|---|---|---|---|
| Acid casein | Examples comp. 1 and 2 | 15% | 1.6% | |
| Presamic casein | Example comp. 3 | 20% | 9.2% | |
| Example 4 | Examples 8A and 9A | 5% | 0.4% | 5.5% |
| Example 5 | Example 8B | 9% | 0.9% | 5.1% |
| Example 6 | Example 8C | 8% | 0.2% | 5.6% |
| Example 7 | Examples 8D and 9B | 10% | 0.8% | 5.2% |

It was thus observed that the analysed commercial dietary caseins reveal a protein impurities content equivalent to 15-20% of the total of the sample protein, while the inorganic impurities content level, defined by means of the value of the determined sulphated ashes, is comprised between 1% and 2.5% by weight in the commercial acid caseins and between 7% and 10% by weight in the presamic caseins, as observable exemplified in the table 1 above.

Thus, a further object of the present invention is also a method for purifying said dietary caseins, leading to the partial, or total, removal of protein impurities, inorganic impurities and/or to the increase of the P/N ratio.

According to the present invention, said dietary casein is treated with water, a polar organic solvent or a mixture thereof; preferably with water.

Preferably said polar organic solvent is a C₁-C₄ alcohol, more preferably methanol, ethanol, isopropanol or a mixture thereof.

According to a first embodiment of the present invention, the selected solvent is used for the purification of casein, in such a manner to dissolve the protein impurities and inorganic impurities to separate them from undissolved casein by means of centrifugation or filtration. Solubility characteristics of casein in the selected solvent as a function of the temperature, pH and/or the addition of additives such as, for example, sodium chloride, calcium chloride and/or ammonium acetate, are used for the implementation of this purification method.

The dietary casein used in the purification process may be directly placed at contact with the selected solvent in the conditions of dissolution of the protein and inorganic impurities and, after a suitable time of contact under stirring, separated from the solution containing the impurities by filtration or centrifugation.

According to a second embodiment of the present invention, the purification shall be performed by first dissolving casein in the selected solvent and subsequently precipitating it by means of suitable variation of pH, temperature and/or addition of additives such as, for example, sodium chloride, calcium chloride and/or ammonium acetate. Even in this case, the separation of casein from the protein and inorganic impurities shall be performed through filtration or centrifugation, such impurities ending up dissolved in the solvent used for purification.

According to the abovementioned first embodiment of the present invention, the dietary casein is placed at contact with the selected solvent, the pH of the mixture is optionally taken to a pH comprised between 3 and 5, or to a pH comprised between 5 and 10. Said pH corrections may be performed for example using aqueous solutions of hydrochloric acid or sodium hydroxide. Said mixture is taken to a temperature comprised between 0°C and 40°C, preferably between 0°C and 10°C. An additive, such as for example sodium chloride, calcium chloride and/or ammonium acetate may be optionally added. The resulting mixture is left under stirring for a period of time comprised between 1 and 24 hours, preferably between 2 and 10 hours.

Said selected solvent is thus used for dissolving the protein impurities and the inorganic impurities and separating them from the undissolved casein, preferably the casein is then separated from the impurities through centrifugation or filtration. Filtration may be of the conventional type or it may be performed by means of membranes of suitable porosity according to the tangential flow filtration method or of the dead end type. According to the abovementioned second embodiment of the present invention, the dietary casein is dissolved in the selected solvent, subsequently subjecting it to precipitation taking it to a pH comprised between 4 and 6, preferably between 4.5 and 5. Any method known to a man skilled in the art may be used for separating casein from the solution containing the protein and inorganic impurities, preferably said casein is separated by means of filtration or centrifugation.

With the aim of obtaining the desired degree of purification, these purification steps may be repeated once or several times. Thus, the same purification method may be repeated, or one may opt to operate purification methods different from each other in sequence.

Disclosed in the present application is a purified dietary casein, obtainable through one of the previously described methods of the present invention, having a protein impurities content preferably lower than 15% by weight, more preferably lower than 10% by weight; an inorganic impurities content preferably lower than 1% by weight, more preferably lower than 0.4% by weight and/or a ratio between the content by weight of phosphorous and the content by weight of nitrogen greater than 5% by weight, more preferably greater than 5.5% by weight.

Also disclosed in the present application is the use of said purified casein, for preparing iron (III) caseinsuccinylate.

Preparation of iron (III) caseinsuccinylate may be performed according to methods known to a man skilled in the art such as, for example, those described in patents IT1150213 and EP939083B1. In an embodiment of the present invention, the method for the preparation of iron (III) caseinsuccinylate described above, comprises the steps of:
a) reacting casein and succinic anhydride in water to obtain the succinylated casein,
b) reacting succinylated casein with iron (III) chloride to obtain iron (III) caseinsuccinylate,
wherein the casein at point a) is purified casein having a low protein impurities and/or inorganic impurities content and/or a high P/N ratio as described above, is performed as follows:
the purified casein is succinylated in water using succinic anhydride keeping the pH during the reaction comprised preferably between 7.5 and 8.5 by adding an aqueous solution of sodium hydroxide.

The reacting temperature is about 20-25°C. At the end of the reaction, the product is precipitated by adding - under stirring - an aqueous solution of hydrochloric acid up to a pH of about 3.

The succinylated casein thus obtained is filtered and dissolved in water by adding an aqueous solution of sodium hydroxide. The solution is filtered to possible undissolved solids; then an aqueous solution of iron (III) chloride is added at the defined amount in function of the content of iron desired in the finished product.

During the addition, there occurs the reduction of the pH and the precipitation of iron (III) caseinsuccinylate.

During the addition of the solution of iron (III) chloride, the pH may be corrected, using an aqueous solution of sodium hydroxide to avoid excessively acidic conditions. In this case, at the end of the addition of iron (III) chloride, precipitation of the complex is completed by adding an aqueous solution of hydrochloric acid.

The formed solid is recovered by filtration and it is suspended in water. In this step, it is possible to add preservatives (methyl and propyl paraben). An aqueous solution of sodium hydroxide is added up to a pH of about 8.5, the undissolved is removed by centrifugation and iron (III) caseinsuccinylate is precipitated by acidification using hydrochloric acid in aqueous solution. The product is dried at low pressure.

Alternatively to drying at low pressure, iron (III) caseinsuccinylate may be obtained from an aqueous solution thereof by means of spray drying.

Said method may also comprise an additional step of microfiltration of the aqueous solution of iron (III) caseinsuccinylate. Said microfiltration step according to the invention is intended to reduce microbial contamination.

Such iron (III) caseinsuccinylate of the invention has improved water solubility characteristics, with respect to that of the prior art; preferably such iron (III) caseinsuccinylate has a solubility in water greater than about 92%. Such solubility value is preferably intended when in 11.5 parts of water.

The calculation of water solubility was performed by solubilising the sample at a pH of about 8.0 using sodium hydroxide with final ratio between water and iron (III) caseinsuccinylate equivalent to 11.5 by weight and filtering the preparation on a filter with porosity equivalent to 6 µm and determining by filter weighing, after drying, the undissolved amount. The percentage solubility of the sample was calculated by subtracting from the weight of the sample itself that of the component left on the filter and comparing the result to the initial weight of the sample. The viscosity of various aqueous preparations was measured at a pH of about 8.0 of the products with concentrations equivalent to 5%, 6% and 8% by weight.

Furthermore, the filtration velocities were measured on membranes with porosity equivalent to 0.45 µm to establish, for the different prepared samples, the possibility of reducing the microbial contamination through microfiltration. This calculation was performed by measuring the time required for passing - by means of an under vacuum system - 5 mL of a solution of the sample in water at a pH of about 8.0 of concentration of 5% by weight through a membrane with a porosity of 0.45 µm of 3 cm².

The table 2 below shows the obtained results.

**Table 2**

| Sample | Solubility in 11.5 parts of water | Solution viscosity (mPa · sec) | | | Filtration time 5 mL solution 5% in water on 0.45 µm (sec) |
|---|---|---|---|---|---|
| | | 5% in water | 6% in water | 8% in water | |
| Ex comp.1 | 89% | 17 | 54 | 715 | ∞ (blocked) |
| Ex comp.2 | 84% | 21 | 67 | 957 | ∞ (blocked) |
| Ex comp. 3 | 85% | 17 | 53 | 863 | 560 |
| Example 8A | 97% | 14 | 29 | 207 | 120 |
| Example 8B | 94% | 13 | 33 | 351 | 150 |
| Example 8C | 98% | 11 | 24 | 195 | 90 |
| Example 8D | 93% | 15 | 44 | 387 | 180 |
| Example 9A | 98% | 12 | 28 | 240 | 180 |
| Example 9B | 92% | 14 | 36 | 411 | 120 |

A further object of the present invention is a composition containing iron (III) caseinsuccinylate of the present invention and at least one physiologically acceptable excipient.

Said composition may be preferably formulated in solid or liquid form, more preferably in liquid form. Said liquid form is preferably a solution or a suspension, more preferably an aqueous solution. Said aqueous solution may be administered orally or parentarally. Said composition comprises an amount of iron (III) caseinsuccinylate according to the invention preferably comprised between 10 and 200 mg of iron, more preferably between 20 and 100 mg of iron, even more preferably about 40 mg, 60 mg or 80 mg of iron.

According to a preferred embodiment of the invention said composition is an aqueous solution comprising about 40 mg, 60 mg or 80 mg of iron dissolved in 15 mL of solution.

Furthermore, it has been surprisingly discovered that the liquid compositions based on iron (III) caseinsuccinylate according to the present invention reveal a considerable reduction of viscosity with respect to the compositions of the prior art.

The viscosity of liquid compositions containing iron (III) caseinsuccinylate of the invention is considerably low with respect to that of the prior art compositions with an equivalent iron value. Such reduction of viscosity in preparations containing iron (III) caseinsuccinylate of the invention, is observable in preparations, obtained starting from iron (III) caseinsuccinylate with an iron content equivalent to 5% and, even more clearly, upon comparison between products with higher iron content, in particular between products with an iron content of 6% and beyond, as observable in table 2. Preferably, the viscosity of a liquid composition containing - in solubilised form - about 8% by weight of iron (III) caseinsuccinylate of the invention is lower than 400 mPa · sec, more preferably lower than 300 mPa · sec.

These surprising characteristics of higher solubility of iron (III) caseinsuccinylate of the invention, and lower viscosity of the liquid commpositions containing them, are particularly useful for the preparation of iron (III) caseinsuccinylate at high concentration of iron (such as, for example equal, or higher, than 40 mg for 15 ml of solution). A preferred solution according to the present invention contains an amount of iron (III) caseinsuccinylate comprised between 4 and 15% by weight, preferably between 4.5 and 12% by weight, more preferably between 5 and 8% by weight, with respect to the total weight.

Such low viscosity characteristic also allows the microfiltration of liquid compositions containing iron (III) caseinsuccinylate of the invention.

This operation is extremely useful for safeguarding the properties of the product, the subsequent formulation and preservation thereof. Having performed the microfiltration operation and reduced the microbial contamination it is also possible to reduce, or even avoid the use of preservatives such as, per example, parabens conventionally used to prevent microbial proliferation.

Actually, this allows considerably reducing the microbial content of the formulations, preferably liquid formulations, containing iron (III) caseinsuccinylate according to the invention.

Up to date, the high viscosity of the liquid formulations (for example, aqueous solutions) based on iron (III) caseinsuccinylate, obtained starting from dietary casein not subjected to a suitable purification process, hindered instead of conducting such microfiltration operation.

The microfiltration according to the invention may occur on membranes with controlled porosity or tangential microfiltration on membranes with controlled porosity with the aim of reducing microbial contamination.

Said microfiltration operation, also linkable to the lower viscosity of the liquid compositions (preferably aqueous solutions) of iron (III) caseinsuccinylate, is particularly useful for preparing oral high shelf life liquid compositions without requiring using questionable high amounts of preservatives.

The following examples have the sole purpose of illustrating some embodiments of the invention and they shall not be deemed restrictive thereto in any manner whatsoever.

### EXAMPLES

### EXAMPLE 1

### Comparative example for the preparation of iron (III) caseinsuccinylate with iron content equivalent to about 5% starting from commercial acid casein.

600 mL of demineralised water are added to 50 g of dietary acid casein, the mixture is stirred for 30 minutes and the pH is taken to a stable pH=8.4 with a 9.5% aqueous solution of sodium hydroxide.

15 g of succinic anhydride are added at small portions maintaining under stirring. During addition, the pH is maintained at about 8.4 by adding the 9.5% sodium hydrochloride solution over time. An aqueous solution of 17% hydrochloric acid is added up to a stable pH =2.8. Isolation is performed by filtering the precipitated succinil casein which is resuspended in 600 Ml of demineralised water. The pH is taken to pH=8.5 using 9.5% aqueous sodium hydroxide, it is kept under stirring, the small undissolved fraction is filtered on a paper filter and, a solution constituted by 14.1 g Iron (III) chloride hexahydrate 119 mL of water is added under stirring. During the addition there is a reduction of the pH and precipitation of Iron (III) caseinsuccinylate. After keeping under stirring for 30 minutes there is pH=2.9 and the product is filtered. The product is suspended in 600mL of water and 5% aqueous sodium hydroxide is added gradually and under stirring up to a stable pH=8.5. Centrifugation is performed to remove the insoluble component equivalent to about 20 g moist; the pH is corrected using hydrochloric acid in aqueous solution at 17% up to pH=2.8 stable. The precipitate is filtered and dried obtaining 54.1 g of iron (III) caseinsuccinylate.

### EXAMPLE 2

### Comparative example for the preparation of iron (III) caseinsuccinylate with iron content equivalent to about 6% starting from commercial acid casein.

600 mL of demineralised water are added to 50 g of dietary acid casein, the mixture is stirred for 30 minutes and the pH is taken to pH=8.4 stable with a 9.5% aqueous solution of sodium hydroxide.

15 g of succinic anhydride are added at small portions maintaining under stirring. During addition the pH is maintained at about 8.4 by adding the 9.5% sodium hydrochloride solution over time. An aqueous solution of 17% hydrochloric acid is added up to a stable pH =2.8. Isolation is performed by filtering the precipitated succinil casein which is resuspended in 600 mL of demineralised water. The pH is taken to pH=8.5 using 9.5% aqueous sodium hydroxide, it is kept under stirring, the small undissolved fraction is filtered on a paper filter and, a solution constituted by 17 g Iron (III) chloride hexahydrate in 150 mL of water is added under stirring. During the addition of the solution of ferric chloride, the pH is maintained in the range comprised between pH=6.0 and pH=6.5 simultaneously adding sodium hydroxide in 5% aqueous solution. After completing the addition of the solution of ferric chloride the pH is corrected to pH=2.8 using 17% aqueous hydrochloric acid. The suspension is kept under stirring for 30 minutes and the product is filtered. The product is suspended in 600 mL of water and 5% aqueous sodium hydroxide is added gradually and under stirring up to stable pH=8.5. Centrifugation is performed to remove the insoluble component equivalent to about 25 g moist; the pH is corrected using hydrochloric acid in aqueous solution al 17% up to stable pH=2.8. The precipitate is filtered and dried obtaining 53.5 g of iron (III) caseinsuccinylate.

### EXAMPLE 3

### Comparative example for the preparation of iron (III) caseinsuccinylate with iron content equivalent to about 5% starting from commercial presamic casein.

Following the method·described in example 1 iron (III) caseinsuccinylate is prepared starting from 50 g of dietary presamic casein. After drying, 54.2 g of iron (III) caseinsuccinylate are obtained.

### EXAMPLE 4

### Purification of commercial acid casein.

6 L of demineralised water are added to 180 g of dietary acid casein, the pH is corrected to pH=7.0 with a 1 M aqueous solution of sodium hydroxide. The mixture is cooled to 4°C, the pH is taken to pH=4.5 with a 1 M aqueous solution of hydrochloric acid. The suspension is kept under stirring for 16 hours and the solid is filtered on buchner.

The moist product is suspended in 6 L of demineralised water, the pH is corrected to pH=7.0 with a 1 M aqueous solution of sodium hydroxide. The mixture is cooled to 4°C, the pH is taken to pH=4.5 with the 1 M aqueous solution of hydrochloric acid. The suspension is kept under stirring for 3 hours and the solid is filtered on buchner.

The moist product is resuspended in 3 L of demineralised water, the pH is corrected to pH=7.0 with the 1 M aqueous solution of sodium hydroxide. The mixture is cooled to 4°C, the pH is taken to pH=4.5 with the 1 M aqueous solution of hydrochloric acid. The suspension is kept under stirring for 3 hours and 390 g of moist solid are filtered on Buchner, the product is dried in a rotary dryer for 20 hours at 25°C and 30 mmHg obtaining 146 g of purified casein.

### EXAMPLE 5

### Purification of commercial acid casein.

6 L of demineralised water are added to 18 g of dietary acid casein, the pH is corrected to pH=7.0 with a 1 M aqueous solution of sodium hydroxide. The mixture is cooled to 4°C, the pH is taken to pH=4.5 with a 1 M aqueous solution of hydrochloric acid. The suspension is kept under stirring for 16 hours and the solid is filtered on buchner.

408 moist grams, which are dried in a rotary dryer for 20 hours at 25°C and 30 mmHg obtaining 162 g of purified casein, are obtained.

### EXAMPLE 6

### Purification of commercial acid casein.

42 L of demineralised water are added to 144 g of dietary acid casein. The pH is corrected to stable pH=7.0 with an 8% solution of sodium hydroxide in water. The pH is taken to pH=3.0 with a solution 0.5 M of aqueous hydrochloric acid. The mixture is cooled to 2°C and the pH is taken to pH=4.0 with aqueous sodium hydroxide 0.01 M. The suspension is kept under stirring for an hour and 318 g of moist solid are filtered on buchner, the product is dried in a rotary dryer for 20 hours at 25°C and 30 mmHg obtaining 126 g of purified casein.

### EXAMPLE 7

### Purification of commercial presamic casein.

6 L of demineralised water are added to 180 g of dietary presamic casein, the mixture is stirred for 30 minutes, it is cooled to 4°C, the mixture is maintained under stirring for 16 hours, the pH is taken to pH=4.5 with a 1 M aqueous solution of hydrochloric acid. The suspension is kept under stirring for an hour and the solid is filtered on buchner.

The moist product is suspended in 6 L of demineralised water, the pH is corrected to pH=7.0 with a 1 M aqueous solution of sodium hydroxide. The mixture is cooled to 4°C, the pH is taken to pH=4.5 with the 1 M aqueous solution of hydrochloric acid. The suspension is kept under stirring for 3 hours and the solid is filtered on buchner.

The moist product is resuspended in 6 L of demineralised water, the pH is corrected to pH=7.0 with the 1 M aqueous solution of sodium hydroxide. The mixture is cooled to 4°C, the pH is taken to pH=4.5 with the 1 M aqueous solution of hydrochloric acid. The suspension is kept under stirring for 16 hours and the solid is filtered on buchner.

290 moist grams, which are dried in a rotary dryer for 20 hours at 25°C and 30 mmHg obtaining 126 g of purified casein, are obtained.

### EXAMPLE 8

### Preparation of iron (III) caseinsuccinylate with iron content equivalent to about 5% starting from the purified caseins as defined in examples 4, 5, 6 and 7.

Following the method described in example 1, iron (III) caseinsuccinylate is prepared starting from the purified caseins obtained as described in examples 4, 5, 6 and 7.

The table below shows the amounts regarding each of the preparations.

**Table 3**

| iron (III) caseinsuccinylate | Raw material | Amount of casein used | Amount of iron (III) caseinsuccinylate obtained |
|---|---|---|---|
| Example 8A | Acid casein purified in Example 4 | 50 g | 54,6 g |
| Example 8B | Acid casein purified in Example 5 | 50 g | 55,2 g |
| Example 8C | Acid casein purified in Example 6 | 50 g | 54,3 g |
| Example 8D | Presamic casein purified in Example 7 | 50 g | 52,7 g |

### EXAMPLE 9

### Preparation of iron (III) caseinsuccinylate with iron content equivalent to about 6% starting from the purified caseins as defined in examples 4 and 7.

Following the method descrive in example 2 iron (III) caseinsuccinylate is prepared starting from the purified caseins obtained as described in examples 4 and 7.

The table below shows the amounts regarding each of the preparations

**Table 4**

| iron (III) caseinsuccinylate | Raw material | Amount of casein used | Amount of iron (III) caseinsuccinylate obtained |
|---|---|---|---|
| Example 9A | Acid casein purified in Example 4 | 50 g | 56.0 g |
| Example 9B | Presamic casein purified in Example 7 | 50 g | 52.5 g |

## Claims

1. Iron (III) caseinsuccinylate **characterised by** a content of iron comprised between 4.5 and 7% by weight, by a solubility greater than about 92% and by having a phosphorous/nitrogen ratio greater than 5% by weight, preferably greater than about 5.5% by weight.

2. Iron (III) caseinsuccinylate according to claim 1, **characterised in that** it contains an amount of protein impurities lower than about 15% by weight, preferably lower than about 10% by weight.

3. Method for preparing iron (III) caseinsuccinylate according to claims I to 2, comprising the following steps:
a. reacting casein and succinic anhydride in water to obtain succinylated casein,
b. reacting succinylated casein with iron (III) chloride to obtain iron (III) caseinsuccinylate,
c. **characterised in that** said casein according to step a) is purified casein, having a protein impurities content lower than 15% by weight and/or inorganic impurities content lower than 1% by weight and/or a phosphorous/nitrogen ratio greater than 5%.

4. Method according to claim 3, **characterised in that** said protein impurities content is lower than about 10% by weight and/or said inorganic impurities content is lower than about 0.4% by weight.

5. Method according to claim 3, **characterised in that** said protein impurities are proteins different from alpha-S1 casein, alpha-S2 casein, beta casein, kappa casein, pseudo-kappa casein.

6. Method according to claim 3, **characterised in that** said purified casein has a phosphorous/nitrogen ratio greater than about 5% by weight.

7. Method according to claim 3, **characterised in that** said purified casein is obtained from acid casein or presamic casein.

8. Method according to claim 3, comprising an additional step of microfiltration of the aqueous solution of iron (III) caseinsuccinylate.

9. Pharmaceutical composition containing iron (III) caseinsuccinylate according to claims 1 to 2 and at least one physiologically acceptable excipient.

10. Composition according to claim 9, **characterised in that** it is in liquid or solid form, preferably in liquid form.

11. Composition according to claim 10, **characterised in that** said liquid form is a solution or a suspension, preferably an aqueous solution.

12. Composition according to claim 9 **characterised in that** said composition is a solution containing about 8% by weight of iron (III) caseinsuccinylate having a viscosity lower than about 400 mPa·sec, preferably lower than about 300 mPa·sec.

13. Composition according to any of claims 9 to 12 wherein iron (III) caseinsuccinylate is contained at an amount comprised between 5 and 15% by weight, preferably between 6 and 12% by weight, more preferably between 7 and 9% by weight, with respect to the total weight of the composition.

14. Composition according to any of claims 9 to 13 for oral or parenteral use, preferably for oral use.

15. Composition according to any of claims 9 to 14, for use in the treatment of iron deficiencies.

16. Composition for use according to claim 15, in the treatment of anemia, pregnancy iron deficiency, malabsorption syndrome, iron deficiency during breastfeeding and iron deficiency during growth.

## Patentansprüche

1. Eisen(III)caseinsuccinylat, **gekennzeichnet durch** einen Gehalt an Eisen, das in einer Menge zwischen 4,5 und 7 Gew.% umfasst ist, eine Löslichkeit von grösser als etwa 92 % und einem Phosphor/Stickstoff-Verhältnis von grösser als 5 Gew.%, vorzugsweise grösser als etwa 5,5 Gew.%.

2. Eisen(III)caseinsuccinylat gemäss Anspruch 1, **dadurch gekennzeichnet, dass** es eine Menge an Protein-Verunreinigungen von geringer als etwa 15 Gew.%, vorzugsweise geringer als etwa 10 Gew.%, enthält.

3. Verfahren zur Herstellung von Eisen(III)caseinsuccinylat gemäss Ansprüchen 1 bis 2, umfassend die nachstehenden Schritte:
a. Umsetzen von Casein und Bernsteinsäureanhydrid in Wasser, um succinyliertes Casein zu erhalten,
b. Umsetzen des succinylierten Caseins mit Eisen(III)chlorid, um Eisen(III)caseinsuccinylat zu erhalten,
c. **dadurch gekennzeichnet, dass** das Casein gemäss Schritt a. gereinigtes Casein ist, das einen Gehalt an Protein-Verunreinigungen von geringer als 15 Gew.% und/oder anorganischen Verunreinigungen von geringer als 1 Gew.% und/oder ein Phosphor/Stickstoff-Verhältnis von grösser als 5 % aufweist.

4. Verfahren gemäss Anspruch 3, **dadurch gekennzeichnet, dass** der Gehalt an Protein-Verunreinigungen geringer als etwa 10 %Gew.% ist und/oder der Gehalt an anorganischen Verunreinigungen geringer als etwa 0,4 Gew.% ist.

5. Verfahren gemäss Anspruch 3, **dadurch gekennzeichnet, dass** die Protein-Verunreinigungen Proteine sind, die von alpha-S1-Casein, alpha-S2-Casein, beta-Casein, kappa-Casein und pseudo-kappa-Casein verschieden sind.

6. Verfahren gemäss Anspruch 3, **dadurch gekennzeichnet, dass** das gereinigte Casein ein Phosphor/Stickstoff-Verhältnis von grösser als etwa 5 Gew.% aufweist.

7. Verfahren gemäss Anspruch 3, **dadurch gekennzeichnet, dass** das gereinigte Casein aus Säure-Casein oder Lab-Casein erhalten wird.

8. Verfahren gemäss Anspruch 3, umfassend einen weiteren Schritt der Mikrofiltrierung der wässrigen Lösung von Eisen(III)caseinsuccinylat.

9. Pharmazeutische Zusammensetzung, enthaltend Eisen(III)caseinsuccinylat gemäss Ansprüchen 1 bis 2 und zumindest einen physiologisch annehmbaren Hilfsstoff.

10. Zusammensetzung gemäss Anspruch 9, **dadurch gekennzeichnet, dass** sie in flüssiger oder fester Form, vorzugsweise in flüssiger Form, vorliegt.

11. Zusammensetzung gemäss Anspruch 10, **dadurch gekennzeichnet, dass** die flüssige Form eine Lösung oder Suspension, vorzugsweise eine wässrige Lösung, ist.

12. Zusammensetzung gemäss Anspruch 9, **dadurch gekennzeichnet, dass** die Zusammensetzung eine Lösung ist, die etwa 8 Gew.% Eisen(III)caseinsuccinylat mit einer Viskosität von weniger als etwa 400 mPa·sek, vorzugsweise weniger als etwa 300 mPa·sek, enthält.

13. Zusammensetzung gemäss irgendeinem der Ansprüche 9 bis 12, wobei das Eisen(III)caseinsuccinylat in einer Menge, umfassend zwischen 5 und 15 Gew.%, vorzugsweise zwischen 6 und 12 Gew.%, stärker bevorzugt zwischen 7 und 9 Gew.%, bezogen auf das Gesamtgewicht der Zusammensetzung, enthalten ist.

14. Zusammensetzung gemäss irgendeinem der Ansprüche 9 bis 13 zur oralen oder parenteralen Verwendung, vorzugsweise zur oralen Verwendung.

15. Zusammensetzung gemäss irgendeinem der Ansprüche 9 bis 14 zur Verwendung bei der Behandlung von Eisenmangel.

16. Zusammensetzung zur Verwendung gemäss Anspruch 15 bei der Behandlung von Anämie, Eisenmangel während der Schwangerschaft, Malabsorptionssyndrom, Eisenmangel während des Stillens und Eisenmangel während des Wachstums.

## Revendications

1. Caséine-succinylate de fer (III) **caractérisé par** une teneur en fer comprise entre 4,5 et 7 % en poids, par une solubilité supérieure à environ 92 % et par un rapport phosphore/azote supérieur à 5 % en poids, de préférence supérieur à environ 5,5 % en poids.

2. Caséine-succinylate de fer (III) selon la revendication 1, **caractérisé en ce qu'**il contient une quantité d'impuretés protéiques inférieure à environ 15 % en poids, de préférence inférieure à environ 10 % en poids.

3. Méthode de préparation de caséine-succinylate de fer (III) selon les revendications 1 à 2, comprenant les étapes suivantes :
a. réaction de la caséine et d'un anhydride succinique dans de l'eau pour obtenir une caséine succinylée,
b. réaction de la caséine succinylée avec du chlorure de fer (III) pour obtenir le caséine-succinylate de fer (III),
c. **caractérisée en ce que** ladite caséine selon l'étape a) est une caséine purifiée, ayant une teneur en impuretés protéiques inférieure à 15 % en poids et/ou une teneur en impuretés inorganiques inférieure à 1 % en poids et/ou un rapport phosphore/azote supérieur à 5 %.

4. Méthode selon la revendication 3, **caractérisée en ce que** ladite teneur en impuretés protéiques est inférieure à environ 10 % en poids et/ou ladite teneur en impuretés inorganiques est inférieure à environ 0,4 % en poids.

5. Méthode selon la revendication 3, **caractérisée en ce que** lesdites impuretés protéiques sont des protéines différentes de la caséine alpha-S1, de la caséine alpha-S2, de la caséine bêta, de la caséine kappa, de la pseudo-caséine kappa.

6. Méthode selon la revendication 3, **caractérisée en ce que** ladite caséine purifiée a un rapport phosphore/azote supérieur à environ 5 % en poids.

7. Méthode selon la revendication 3, **caractérisée en ce que** ladite caséine purifiée est obtenue à partir d'une caséine acide ou d'une caséine présamique.

8. Méthode selon la revendication 3, comprenant une étape supplémentaire de microfiltration de la solution aqueuse du caséine-succinylate de fer (III).

9. Composition pharmaceutique contenant le caséine-succinylate de fer (III) selon les revendications 1 à 2 et au moins un excipient physiologiquement acceptable.

10. Composition selon la revendication 9, **caractérisée en ce qu'**elle est sous forme liquide ou solide, de préférence sous forme liquide.

11. Composition selon la revendication 10, **caractérisée en ce que** ladite forme liquide est une solution ou une suspension, de préférence une solution aqueuse.

12. Composition selon la revendication 9 **caractérisée en ce que** ladite composition est une solution contenant environ 8 % en poids de caséine-succinylate de fer (III) ayant une viscosité inférieure à environ 400 mPa.s, de préférence inférieure à environ 300 mPa.s.

13. Composition selon l'une quelconque des revendications 9 à 12 dans laquelle le caséine-succinylate de fer (III) est contenu en une quantité comprise entre 5 et 15 % en poids, de préférence entre 6 et 12 % en poids, plus préférablement entre 7 et 9 % en poids, par rapport au poids total de la composition.

14. Composition selon l'une quelconque des revendications 9 à 13 pour un usage oral ou parentéral, de préférence pour un usage oral.

15. Composition selon l'une quelconque des revendications 9 à 14, pour son utilisation dans le traitement des carences en fer.

16. Composition pour son utilisation selon la revendication 15, dans le traitement de l'anémie, d'une carence en fer durant la grossesse, du syndrome de malabsorption, de la carence en fer durant l'allaitement et de la carence en fer durant la croissance.
